# EUROPÄISCHE PATENTSCHRIFT

(11) **EP 1 776 055 B1**
(45) Veröffentlichungstag und Bekanntmachung des Hinweises auf die Patenterteilung: **01.02.2012**
(21) Anmeldenummer: 05770477.7
(22) Anmeldetag: 02.07.2005
(51) Int. Cl.: A61B 17/80

(54) **FIXATIONSSYSTEM FÜR KNOCHEN UND FÜLLKÖRPER FÜR EIN FIXATIONSSYSTEM FÜR KNOCHEN**
BONE-FIXATION SYSTEM AND FILLER ELEMENT FOR BONE-FIXATION SYSTEM
SYSTEME DE FIXATION D'OS ET MATERIAU DE REMPLISSAGE POUR SYSTEME DE FIXATION D'OS

(30) Priorität: 19.07.2004 DE 102004035546
(43) Veröffentlichungstag der Anmeldung: 25.04.2007
(73) Patentinhaber: Wolter, Dietmar, 22955 Hoisdorf (DE)
(72) Erfinder: Wolter, Dietmar, 22955 Holsdorf (DE)
(74) Vertreter: Glawe, Delfs, Moll
(86) Internationale Anmeldenummer: PCT/EP2005/007164
(87) Internationale Veröffentlichungsnummer: WO 2006/007965

(56) Entgegenhaltungen:
- US-A1- 2002 183 756
- US-A1- 2003 225 409

## Beschreibung

Die Erfindung bezieht sich auf ein Fixationssystem für Knochen mit einem Verbindungsträger mit mehreren Durchgangslöchern und wenigstens einer in ein Durchgangsloch einsetzbaren Knochenschraube.

Für die Stabilisierung gebrochener Knochen oder von Knochenabschnitten, welche aufgrund von Fehlheilungen oder im Rahmen von operativen Durchtrennungen instabil sind, wird seit 1886 einem Vorschlag von C. Hansmann folgend eine Platte aus Metall mit Durchgangslöchern und Knochenschrauben verwendet. Dieses System hat sich weltweit durchgesetzt. Es ist heute neben der Marknagelung und der Osteosynthese mittels Fixateur extern die dritte wichtig Osteosynthesetechnik.

Die Dimensionierung von Platte und Knochenschrauben, die richtig Auswahl der Metalle für Platte und Knochenschrauben im Hinblick auf ihre mechanischen Eigenschaften und ihrer Biokompatibilität sind seit dieser Zeit intensiv von vielen Forschergruppen untersucht worden.

Die Bedeutung der Kraftschlüssigkeit zwischen Knochenschraube und Platte ist in den vergangenen 20 Jahren zunehmend erkannt und umgesetzt worden. In den letzten Jahren haben eigene Forschungen ergeben, daß bei einem Fixationssystem mit Platte und winkelstabil befestigten (d.h. nicht in der Platte schwenkbaren) Schrauben - also einer kraftschlüssigen Verbindung von Knochenschraube und Platte - die Belastung der Platte auf Höhe der ersten, dem instabilen Knochenabschnitt benachbarten Knochenschraube am höchsten ist. Danach fällt die Lastübertragung von Durchgangsloch zu Durchgangsloch weiter ab. In etwa überträgt die Knochenschraube im ersten Durchgangsloch ca. 50 bis 60 % der Kräfte, die zweite Knochenschraube etwa 20 bis 30 % und die dritte Knochenschraube etwa 10 bis 20 %.

Um dieser Tatsache zu entsprechen, wurde die Festigkeit der Platte durch angepaßte Verbreiterungen oder Verdickungen der Platte auf Höhe der einzelnen Löcher verändert. Dies ist in der WO 01/19264 A beschrieben. Diese Lösung kompensiert die Schwächung der Platte durch das Durchgangsloch. Dennoch besteht ein Bedürfnis, die Festigkeit der Platte weiter zu verbessern.

Ferner werden weiterhin Platten ohne Verbreiterungen oder Verdickungen verwendet. Insbesondere im Rahmen der Versorgung kleinerer Knochen, z.B. im Bereich des Handskeletts, sind aufgrund der anatomischen Gegebenheiten solche Verstärkungen der Platten im Bereich der besonders beanspruchten Durchgangslöcher nicht vorteilhaft. Deshalb besteht ein Bedürfnis, die Festigkeit von Platten ohne Verbreiterungen oder Verdickungen auf andere Weise zu steigern.

Dies gilt entsprechend für andere Fixationssysteme, beispielsweise für Marknägel.

Die US 2002/0183756 A1 beschreibt ein Plattensystem für die Wirbelsäule mit einer Platte mit mehreren Durchgangslöchern und wenigstens einer in ein Durchgangsloch einsetzbaren Knochenschraube. In ein Durchgangsloch ist ein Sicherungsteil einsetzbar, dass der Sicherung einer Knochenschraube dient.

Davon ausgehend liegt der Erfindung die Aufgabe zugrunde, ein Fixationssystem mit weiter oder auf andere Weise verbesserter Festigkeit zur Verfügung zu stellen oder zu ermöglichen.

Die Aufgabe wird durch ein Fixationssystem mit den Merkmalen des Anspruches 1 gelöst. Vorteilhafte Ausgestaltungen des Fixationssystems sind in den Unteransprüchen angegeben.

Das erfindungsgemäße Fixationssystem für Knochen mit einem Verbindungsträger mit mehreren Durchgangslöchern und wenigstens einer in ein Durchgangsloch einsetzbaren Knochenschraube weist wenigstens einen in ein Durchgangsloch einsetzbaren Füllkörper und Mittel zum Festlegen des Füllkörpers im Durchgangsloch auf, wobei der Füllkörper ein Übermaß bezüglich des Durchgangsloches aufweist.

Der erfindungsgemäße Füllkörper für ein Fixationssystem für Knochen mit einem Verbindungsträger mit mehreren Durchgangslöchern und wenigstens einer in ein Durchgangsloch einsetzbaren Knochenschraube ist in ein Durchgangsloch des Verbindungsträgers einsetzbar und weist Mittel zum Festlegen im Durchgangsloch auf.

Im Rahmen der Erfindung wurde erkannt, daß in vielen Fällen eine Einbuße der Festigkeit von Fixationssystemen daraus resultiert, daß ein Durchgangsloch eines Verbindungsträgers nicht durch eine Knochenschraube besetzt wird. Die Nichtbesetzung von Durchgangslöchern erfolgt vielfach deshalb, weil die Knochenschraube störend oder überflüssig ist. Dies passiert regelhaft besonders bei langstreckigen Brüchen oder bei der Versorgung kleinerer Knochen mittels Platten oder anderen Verbindungsträgern ohne Verbreiterungen oder Verdickungen. Die Schwächung ist sowohl bei Verbindungsträgern mit als auch bei Verbindungsträgern ohne solche Verstärkungen um das Durchgangsloch gegeben, ist jedoch bei Platten ohne solche Verstärkungen besonders problematisch. Erfindungsgemäß wird nun ein nicht mit einer Knochenschraube besetztes Durchgangsloch mit einem Füllkörper aufgefüllt, der entsprechend dem Schraubenkopf einer Knochenschraube die Festigkeit dieses Abschnittes des Verbindungsträgers erhöht. Durch Auffüllen mit einem Füllkörper wird an Durchgangslöchern, die nicht mit einer Knochenschraube besetzt werden, im wesentlichen dieselbe Festigkeit wie bei eingesetzter Knochenschraube erzielt und die Festigkeit des Fixationssystems insgesamt verbessert. Die Verbesserung betrifft sowohl Fixationssysteme mit als auch Fixationssysteme ohne Verbreiterung oder Verdickung des Verbindungsträgers. Das Einsetzen und Festlegen des Füllkörpers im Durchgangsloch kann außerhalb oder innerhalb des menschlichen Körpers erfolgen, sobald entschieden ist, welche Durchgangslöcher mit Knochenschrauben bestückt werden und welche nicht.

Das Ausfüllen des Durchgangsloches durch einen Füllkörper führt grundsätzlich immer zu einer Erhöhung der Festigkeit dieses Abschnittes des Verbindungsträgers, unabhängig von der Ausgestaltung der Mittel zum Festlegen des Füllkörpers im Durchgangsloch. Besonders große Steigerungen der Festigkeit werden erzielt, wenn der Füllkörper kraftschlüssig an der Wand des Durchgangsloches befestigt ist, z.B. durch Gewindeverbindung oder durch Materialumformung. Gemäß einer Ausgestaltung weist deshalb der Füllkörper ein Außengewinde und das Durchgangsloch ein Innengewinde auf, in das der Füllkörper mit seinem Außengewinde einschraubbar ist. Dabei sind die Mittel zum Festlegen vom Außengewinde des Füllkörpers und vom Innengewinde des Durchgangsloches gebildet. Gemäß einer anderen Ausgestaltung mit kraftschlüssiger Befestigung des Füllkörpers an der Wand des Durchgangsloches hat der Füllkörper ein in das Durchgangsloch einformbares Außengewinde und/oder das Durchgangsloch ein in den Füllkörper einformbares Innengewinde. Bei dieser Ausgestaltung sind die Mittel zum Festlegen von dem einformbaren Außengewinde und/oder dem einformbaren Innengewinde gebildet. Dabei sind der Füllkörper und das Durchgangsloch z.B. so ausgeführt, wie in der DE 43 43 117 C2 oder der EP 1 143 867 B1 für verschiedene Ausführungen des Schraubenkopfes und des Durchgangsloches von Fixationssystemen im einzelnen beschrieben. Die diesbezüglichen Angaben in den beiden vorerwähnten Druckschriften sind in die vorliegende Anmeldung einbezogen. Bei beiden vorbeschriebenen Ausgestaltungen ist eine Erhöhung der Festigkeit der Platte in dem Bereich des aufgefüllten Durchgangsloches um etwa 70 bis 100 % erreichbar.

Einbezogen sind auch Ausführungen, bei denen der Füllkörper ein Außengewinde und das Durchgangsloch ein Innengewinde aufweist, die sowohl ohne Umformung ineinanderschraubbar als auch unter Umformung und Zerstörung mindestens einen Gewindes miteinander verbindbar sind.

Der Füllkörper hat ein Übermaß bezüglich des Durchgangsloches. Bei dieser Ausgestaltung sind die Mittel zum Festlegen durch das Übermaß des Füllkörpers im Durchgangsloch gebildet. Z. B. durch Einpressen des Füllkörpers, Aufschrumpfen des Verbindungsträgers oder dgl. ist der Füllkörper im Durchgangsloch festlegbar.

Die Ausgestaltungen mit einformbarem Außengewinde des Füllkörpers und/oder Übermaß des Füllkörpers sind insbesondere für Fixationssysteme (nachfolgend "konventionelle Fixationssysteme") geeignet, bei denen der Schraubenkopf der Knochenschraube keine feste Verbindung mit der Wand des Durchgangsloches aufweist. Geeignet für diese konventionellen Fixationssysteme ist auch eine weitere Ausgestaltung, bei der der Füllkörper mehrere Füllkörperteile und eine diese überbrückende Schraube zum Verklemmen des Füllkörpers im Durchgangsloch durch Zusammenziehen der Füllkörperteile mittels der Schraube aufweist. Hierbei sind die Mittel zum Festlegen durch die mehreren Füllkörperteile und die diese überbrückende Schraube gebildet.

Diese Ausgestaltung ist auch für das Auffüllen länglicher Durchgangslöcher geeignet, welche zur Kompression des Bruchspaltes dienen. Dabei ist die Wand des Durchgangsloches so gestaltet, daß eine schiefe Ebene vorhanden ist. Beim Eindrehen der Knochenschraube wandert der sphärische Schraubenkopf die schiefe Ebene herab und nimmt das damit verbundene Knochenfragment mit, so daß die Knochenfragmente im Bruchspalt komprimiert werden. Für das Auffüllen von länglichen Durchgangslöchern geeignet sind auch entsprechend geformte Füllkörper mit einem Übermaß bezüglich des Durchgangsloches.

Gemäß einer Ausgestaltung ist der Füllkörper ein Spreizkörper und einen darin einsetzbaren Spreizkern zum Spreizen und Verklemmen des Spreizkörpers im Durchgangsloch. Dabei sind die Mittel zum Festlegen durch den Spreizkörper und den darin einsetzbarem Spreizkern gebildet. Dieser Füllkörper wird ähnlich einem Dübel in das Durchgangsloch eingebracht und in diesem befestigt. Er ist für winkelstabile und für konventionelle Fixationssysteme geeignet.

Gemäß einer Ausgestaltung besteht der Füllkörper ganz oder teilweise aus einem Material mit Memoryeffekt, das durch Wärmezufuhr auf seine Ausgangsform ausdehnbar ist, mit der es einen Klemmsitz im Durchgangsloch und/oder am Verbindungsträger und/oder eine formschlüssige Verbindung mit dem Durchgangsloch und/oder mit dem Verbindungsträger aufweist. Somit ist der Füllkörper durch Klemmung und/oder Formschluß im Durchgangsloch festgelegt. Dieser Füllkörper ist sowohl für winkelstabile als auch für konventionelle Fixationssysteme geeignet.

Der Klemmsitz im Durchgangsloch kann durch Aufweitung des Umfanges des Füllkörpers im Durchgangsloch bewirkt werden. Die formschlüssige Verbindung kann durch Aufweitung des Füllkörpers bis zur Anlage an zwei Fasen an den beiden Enden des Durchgangsloches oder durch Übergreifen der Außenseiten des Verbindungsträgers erfolgen.

Das Material mit Memoryeffekt ist z.B. ein Metall oder ein Kunststoff. Die Wärmezufuhr kann z.B. durch die Körperwärme des Patienten erfolgen oder durch eine gezielte lokale Erwärmung mittels einer Heiz- bzw. Strahlungsvorrichtung.

Gemäß einer Ausgestaltung ist der Füllkörper von einem im fließfähigen Zustand in das Durchgangsloch einfüllbaren und im Plattenloch zu einem festen Füllkörper aushärtbaren Material gebildet. Das ausgehärtete Material ist z.B. ein Metall oder ein Kunststoff (z.B. Polymethacrylat). Dieser Füllkörper ist durch Verkleben und/oder Klemmen und/oder Formschluß im Durchgangsloch festgelegt. Er ist sowohl für winkelstabile als auch für konventionelle Fixationssysteme geeignet.

Gemäß einer Ausgestaltung weist/weisen der Füllkörper und/oder das Durchgangsloch aufgerauhte oder anderweitige Kontaktflächen mit erhöhten Reibbeiwert auf. Durch Reibschluß wird die Festlegung des Füllkörpers im Durchgangsloch unterstützt oder bewirkt.

Die folgenden drei Ausgestaltungen steigern besonders die Festigkeit im Bereich des Durchgangsloches: Gemäß einer Ausgestaltung entsprechen die Form und die Abmessungen des Füllkörpers im wesentlichen der Form und den Abmessungen des Durchgangsloches, so daß er das Durchgangsloch im wesentlichen ausfüllt. Gemäß einer weiteren Ausgestaltung sind die Mittel zum Festlegen im wesentlichen über die gesamte Wand des Durchgangsloches erstreckt. Gemäß einer weiteren Ausgestaltung besteht der Füllkörper aus einem Material, das im wesentlichen dieselbe Festigkeit wie das Material des Verbindungsträgers aufweist. Vorzugsweise gilt dies auch für die Festigkeit des Materials der Knochenschraube.

Gemäß einer Ausgestaltung ist der Füllkörper aus Metall und/oder Kunststoff. Gemäß einer weiteren Ausgestaltung ist der Füllkörper aus Titan oder einer Titanlegierung.

Gemäß einer Ausgestaltung ist der Verbindungsträger eine Platte. Gemäß einer anderen Ausgestaltung ist der Verbindungsträger ein Marknagel.

Gemäß einer weiteren Ausgestaltung ist der Verbindungsträger und/oder die Knochenschraube aus Titan, einer Titanlegierung oder aus einem anderen Metall.

Schließlich ist gemäß einer Ausgestaltung der Füllkörper insgesamt oder zumindest in der Kontaktzone zum Verbindungsträger aus einem härteren Material als der Verbindungsträger oder zumindest die Kontaktzone des Verbindungsträgers zum Füllkörper oder umgekehrt. Die Paarung von härterem Material und weicherem Material zumindest für die Kontaktzonen von Füllkörper und Verbindungsträger ist insbesondere vorteilhaft für Mittel zum Festlegen, die ein formendes Gewinde aufweisen, welches aus dem härteren Material besteht.

Nachfolgend wird die Erfindung anhand der anliegenden Zeichnung von Ausführungsbeispielen näher erläutert. In der Zeichnung zeigen:
- Fig. 1: einen Füllkörper in einem Durchgangsloch mit einer umformbaren Lippe in einem vertikalen Teilschnitt;
- Fig. 2: einen Füllkörper in einem Durchgangsloch mit einem stärker eingeformten Innengewinde in einem vertikalen Teilschnitt;
- Fig. 3: einen Füllkörper in einem Durchgangsloch mit Innengewinde in einem vertikalen Teilschnitt;
- Fig. 4: einen als Spreizkörper ausgebildeten Füllkörper in einem Durchgangsloch in einem vertikalen Teilschnitt;
- Fig. 5: einen Füllkörper mit zwei Füllkörperteilen und eine diesen überbrückende Schraube in einem Durchgangsloch in einem vertikalen Teilschnitt.

Bei der nachfolgenden Erörterung verschiedener Ausführungsbeispiele sind übereinstimmende oder im wesentlichen übereinstimmende Merkmale mit denselben Bezugsziffern bezeichnet, die zur Unterscheidung der Ausführungsbeispiele mit Anstrichen versehen sind.

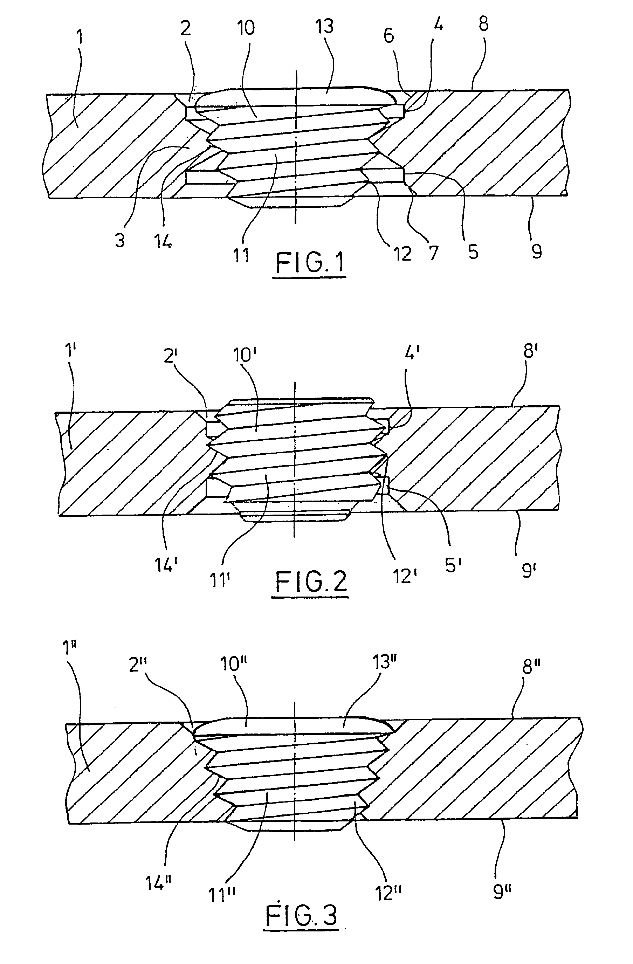

Gemäß Fig. 1 hat eine Platte 1 ein quer gerichtetes Durchgangsloch 2, in dem etwa mittig eine umformbare Lippe 3 angeordnet ist. An die Lippe 3 grenzen Zylinderabschnitte 4, 5 des Durchgangsloches 2 an. Ausgehend von den äußeren Enden der Zylinderabschnitte 4, 5 ist das Durchgangsloch 2 durch Fasen 6, 7 zur Oberseite 8 und zur Unterseite 9 der Platte 1 hin erweitert.

Im Durchgangsloch 2 ist ein Füllkörper 10 angeordnet, der einen im wesentlichen konischen Gewindeabschnitt 11 mit einem Außengewinde 12 aufweist. Der Füllkörper 10 weist an dem Ende des konischen Gewindeabschnittes 11 mit dem größeren Durchmesser einen umlaufenden Ringwulst 13 auf.

An der Oberseite weist der Füllkörper einen - nicht sichtbaren - Werkzeugeingriff auf, z.B. einen Schlitz für das Einsetzen einer Schraubendreherklinge.

Der Füllkörper 10 und die Platte 1 sind z.B. aus Titan oder einer Titanlegierung. Bevorzugt ist der Füllkörper 10 aus einem härteren Material als die Platte 1.

Der Füllkörper 10 wird mit dem Ende des konischen Gewindeabschnittes 11 mit dem kleineren Durchmesser voran in das Durchgangsloch 2 eingedreht. Dabei formt das Außengewinde 12 in die Lippe 3 ein Innengewinde 14 ein. Die Umformkraft steigt allmählich an, wobei der Füllkörper 10 immer fester im Durchgangsloch 2 sitzt. In der gezeigten Lage stabilisiert der Füllkörper 10 die Platte 1 im Bereich des Durchgangsloches 2.

Die Ausführung von Fig. 2 unterscheidet sich von der vorbeschriebenen im wesentlichen dadurch, daß der Formkörper 10' einen annähernd zylindrischen Gewindeabschnitt 11' mit leichter Verringerung des Durchmessers zu beiden Enden hin aufweist. Der Außendurchmesser des Außengewindes 12` unterschreitet überall nur geringfügig den Innendurchmesser der Zylinderabschnitte 4', 5' des Durchgangsloches 2' der Platte 1'. Infolgedessen kommt es beim Eindrehen des Füllkörpers 10' zur Einformung eines ausgeprägteren Innengewindes 14' in der Lippe 3` als bei Fig. 1. Die Festigkeit der Platte 1' im Bereich des Durchgangsloches 2' ist hierdurch weiter erhöht.

Die Verringerung des Außendurchmessers des Gewindes 12' zu den beiden Enden hin erleichtert das Einfädeln des Füllkörpers 10' in das Durchgangsloch 2' und das Einformen des Innengewindes 14' in der Anfangsphase.

Gemäß Fig. 3 ist in einer Platte 1" ein Durchgangsloch 2" vorhanden, das sich von der Oberseite 8" zur Unterseite 9" hin verjüngt. In dem Durchgangsloch 2" ist ein sich ebenfalls allmählich verjüngendes Innengewinde 14" von vornherein vorhanden.

In die Platte 1" eingedreht ist ein Füllkörper 10", der mit dem Füllkörper 10 von Fig. 1 übereinstimmt. Das Außengewinde 12" des Füllkörpers 10" ist komplementär zum Innengewinde 14".

Der Füllkörper 10" wird in das Durchgangsloch 2" eingeschraubt und aufgrund der Konizität von Außengewinde 12" und Innengewinde 14" in diesem verklemmt. Da der Füllkörper 10" annähernd über die gesamte Wand des Durchgangsloches 2" mit der Platte 1" verbunden bzw. verklemmt ist, ist die Festigkeit im Bereich des Durchgangsloches 2" besonders erhöht.

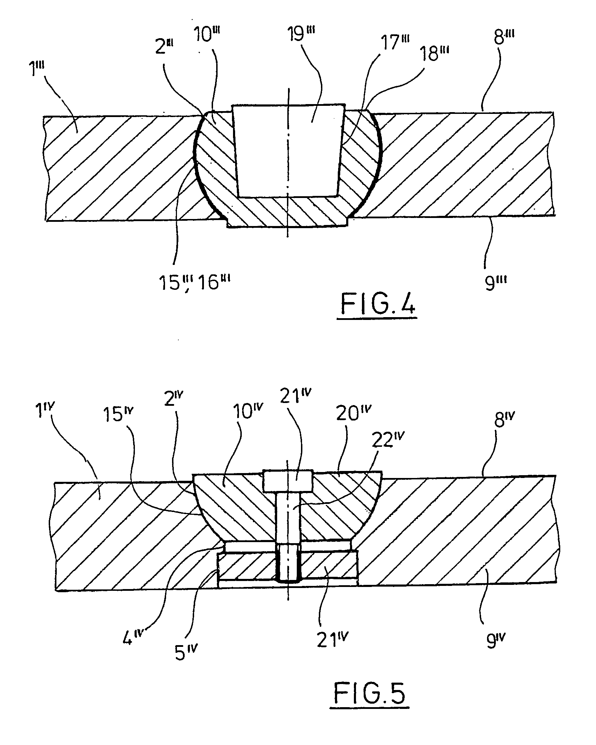

Gemäß Fig. 4 weist eine Platte 1''' ein Durchgangsloch 2''' mit einer kugelkalottenförmigen Wand 15''' auf. In das Durchgangsloch 2''' ist ein als Spreizkörper ausgebildeter Füllkörper 10''' eingesetzt. Der Füllkörper 10''' hat einen kugelkalottenförmigen Mantel 16'''.

Ferner weist der Füllkörper 10''' ein Sackloch 17''' auf, das zu seiner Oberseite 18''' hin geöffnet ist und sich zu dieser hin konisch erweitert.

Im Bereich des Sackloches 17''' ist der Füllkörper 10''' in Axialrichtung geschlitzt. In das Sackloch 17''' ist ein konischer Spreizkern 19''' eingeschraubt. Hierzu hat das Sackloch 17''' ein Innengewinde und der Spreizkern 19''' ein Außengewinde, die ineinandergreifen. Durch Einschrauben des Spreizkerns 19''' in das Sackloch 17''' wird der Füllkörper 10''' im Durchgangsloch 2''' gleichmäßig verspannt und die Festigkeitssteigerung in der Platte 1''' bewirkt.

Gemäß Fig. 5 hat eine Platte 1^{IV} ein Durchgangsloch 2^{IV} mit einer oberen, kalottenförmigen Wand 15^{IV}. einem daran angrenzenden Zylinderabschnitt 4^{IV} und einem daran angrenzenden weiteren Zylinderabschnitt 5^{IV}, dessen Innendurchmesser den des vorerwähnten Zylinderabschnittes 4^{IV} übersteigt.

Der Füllkörper 10^{IV} hat ein kalottenförmiges Füllkörperteil 20^{IV}, das an der Wand 15^{IV} anliegt, und ein zylindrisches Füllkörperteil 21^{IV}, das an dem weiteren Zylinderabschnitt 5^{IV} anliegt. Die Füllkörperteile 20^{IV}, 21^{IV} sind durch eine mit einem Schraubenkopf 22^{IV} an der Oberseite des Füllkörperteils 20^{IV} abgestützte, axial durch dieses hindurchgeführte und in das Füllkörperteil 21^{IV} hineingeschraubte Schraube 23^{IV} miteinander verspannt und mit der Durchgangsbohrung 2^{IV} verklemmt. Hierdurch wird die Festigkeit im Bereich des Durchgangsloches 2^{IV} gesteigert.

## Patentansprüche

1. Fixationssystem für Knochen mit einem Verbindungsträger (1) mit mehreren Durchgangslöchern (2) und wenigstens einer in ein Durchgangsloch (2) einsetzbaren Knochenschraube, das wenigstens einen in ein Durchgangsloch einsetzbaren Füllkörper (10) und Mittel zum Festlegen des Füllkörpers im Durchgangsloch (2) aufweist, **dadurch gekennzeichnet, daß** der Füllkörper (10) ein Übermaß bezüglich des Durchgangslochs (2) aufweist, so daß beim Einsetzen des Füllkörpers (10) in das Durchgangsloch (2) eine Materialumformung stattfindet.

2. Fixationssystem nach Anspruch 1, **dadurch gekennzeichnet, daß** der Füllkörper (10) ein Außengewinde (12) und das Durchgangsloch ein Innengewinde (14) aufweisen, in das der Füllkörper (10) mit seinem Außengewinde (12) einschraubbar ist.

3. Fixationssystem nach Anspruch 1 oder 2, **dadurch gekennzeichnet, daß** der Füllkörper (10) ein in das Durchgangsloch (2) einformbares Außengewinde (12) und/oder das Durchgangsloch (2) ein in den Füllkörper einformbares Innengewinde (14) aufweist/aufweisen.

4. Fixationssystem nach einem der Ansprüche 1 bis 3, **dadurch gekennzeichnet, daß** der Füllkörper (10) und/oder das Durchgangsloch (2) aufgerauhte oder anderweitige Kontaktflächen mit erhöhtem Reibbeiwert aufweist/aufweisen.

5. Fixationssystem nach einem der Ansprüche 1 bis 4, **dadurch gekennzeichnet, daß** die Form und die Abmessungen des Füllkörpers (10) der Form und den Abmessungen des Durchgangsloches (2) entsprechen.

6. Fixationssystem nach einem der Ansprüche 1 bis 5, **dadurch gekennzeichnet, daß** der Füllkörper (10) aus einem Material ist, das im wesentlichen dieselbe Festigkeit wie das Material des Verbindungsträgers (1) aufweist.

7. Fixationssystem nach einem der Ansprüche 1 bis 6, **dadurch gekennzeichnet, daß** der Füllkörper (10) aus Metall und/oder aus Kunststoff ist.

8. Fixationssystem nach einem der Ansprüche 1 bis 7, **dadurch gekennzeichnet, daß** der Fullkörper (10) aus Titan oder einer Titanlegierung ist.

9. Fixationssystem nach einem der Ansprüche 1 bis 8, **dadurch gekennzeichnet, daß** der Verbindungsträger eine Platte (1) ist.

10. Fixationssystem nach einem der Ansprüche 1 bis 9, **dadurch gekennzeichnet, daß** der Verbindungsträger ein Marknagel ist.

11. Fixationssystem nach einem der Ansprüche 1 bis 10, **dadurch gekennzeichnet, daß** der Verbindungsträger (1) und/oder die Knochenschraube aus Titan, einer Titanlegierung oder aus einem anderen Metall ist.

12. Fixationssystem nach einem der Ansprüche 1 bis 11, **dadurch gekennzeichnet, daß** der Füllkörper (10) insgesamt oder zumindest in der Kontaktzone zum Verbindungsträger (1) aus einem härteren Material als der Verbindungsträger (1) oder zumindest die Kontaktzone des Verbindungsträgers (1) zum Füllkörper (10) ist oder umgekehrt,

## Claims

1. Fixation system for bones, comprising a connecting support (1), with a plurality of through-holes (2), and at least one bone screw insertable into a through-hole (2), which fixation system has at least one filler body (10), insertable into a through-hole, and means for fastening the filler body in the through-hole (2), **characterized in that** the filler body (10) has an overdimension in relation to the through-hole (2), such that a material deformation takes place when the filler body (10) is inserted into the through-hole (2).

2. Fixation system according to Claim 1, **characterized in that** the filler body (10) has an external thread (12), and the through-hole has an internal thread (14) into which the filler body (10) is screwable with its external thread (12).

3. Fixation system according to Claim 1 or 2, **characterized in that** the filler body (10) has an external thread (12) shapeable into the through-hole (2) and/or the through-hole (2) has an internal thread (14) shapeable into the filler body.

4. Fixation system according to one of Claims 1 to 3, **characterized in that** the filler body (10) and/or the through-hole (2) have/has roughened or other contact surfaces with an increased coefficient of friction.

5. Fixation system according to one of Claims 1 to 4, **characterized in that** the shape and the dimensions of the filler body (10) correspond to the shape and the dimensions of the through-hole (2).

6. Fixation system according to one of Claims 1 to 5, **characterized in that** the filler body (10) is made from a material that has substantially the same strength as the material of the connecting support (1) .

7. Fixation system according to one of Claims 1 to 6, **characterized in that** the filler body (10) is made from metal and/or of plastic.

8. Fixation system according to one of Claims 1 to 7, **characterized in that** the filler body (10) is made from titanium or a titanium alloy.

9. Fixation system according to one of Claims 1 to 8, **characterized in that** the connecting support is a plate (1).

10. Fixation system according to one of Claims 1 to 9, **characterized in that** the connecting support is an intramedullary nail.

11. Fixation system according to one of Claims 1 to 10, **characterized in that** the connecting support (1) and/or the bone screw is made from titanium, a titanium alloy or another metal.

12. Fixation system according to one of Claims 1 to 11, **characterized in that** the filler body (10) in its entirety, or at least in the area of contact to the connecting support (1), is made from a harder material than the connecting support (1) or at least than the area of contact of the connecting support (1) to the filler body (10), or vice versa.

## Revendications

1. Système de fixation destiné aux os, comportant un support d'assemblage (1) avec plusieurs trous débouchants (2) et au moins une vis pouvant être introduite dans le trou débouchant (2), lequel comporte au moins un corps de remplissage (10) pouvant être inséré dans le trou débouchant, et des moyens pour fixer le corps de remplissage dans le trou débouchant (2), **caractérisé en ce que** le corps de remplissage (10) est surdimensionné par rapport au trou débouchant (2), de telle sorte qu'une déformation de matière se produit au moment de l'introduction du corps de remplissage (10) dans le trou débouchant (2).

2. Système de fixation selon la revendication 1, **caractérisé en ce que** le corps de remplissage (10) comporte un filetage extérieur (12) et le trou débouchant (2) comporte un filetage intérieur (14), dans lequel le corps de remplissage (10) peut être vissé avec son filetage extérieur (12).

3. Système de fixation selon la revendication 1 ou 2, **caractérisé en ce que** le corps de remplissage (10) comporte un filetage extérieur (12) pouvant s'imprimer dans le trou débouchant (2) et/ou le trou débouchant (2) comporte un filetage intérieur (14) pouvant s'imprimer dans le corps de remplissage (10).

4. Système de fixation selon l'une quelconque des revendications 1 à 3, **caractérisé en ce que** le corps de remplissage (10) et/ou le trou débouchant (2) a/ont des surfaces de contact rendues rugueuses ou des surfaces de contact avec un coefficient de frottement élevé obtenu d'une autre manière.

5. Système de fixation selon l'une quelconque des revendications 1 à 4, **caractérisé en ce que** la forme et les dimensions du corps de remplissage (10) correspondent à la forme et aux dimensions du trou débouchant (2).

6. Système de fixation selon l'une quelconque des revendications 1 à 5, **caractérisé en ce que** le corps de remplissage (10) est réalisé dans un matériau qui possède sensiblement la même résistance que le matériau du support d'assemblage (1).

7. Système de fixation selon l'une quelconque des revendications 1 à 6, **caractérisé en ce que** le corps de remplissage (10) est réalisé en métal et/ou en matière plastique.

8. Système de fixation selon l'une quelconque des revendications 1 à 7, **caractérisé en ce que** le corps de remplissage (10) est réalisé en titane ou dans un alliage de titane.

9. Système de fixation selon l'une quelconque des revendications 1 à 8, **caractérisé en ce que** le support d'assemblage est une plaque (1).

10. Système de fixation selon l'une quelconque des revendications 1 à 9, **caractérisé en ce que** le support d'assemblage est un clou médullaire.

11. Système de fixation selon l'une quelconque des revendications 1 à 10, **caractérisé en ce que** le support d'assemblage (1) et/ou la vis sont réalisés en titane, dans un alliage de titane ou dans un autre métal.

12. Système de fixation selon l'une quelconque des revendications 1 à 11, **caractérisé en ce que** le corps de remplissage (10) en totalité ou au moins dans la zone de contact avec le support d'assemblage (1) est réalisé dans un matériau plus dur que le support d'assemblage (1) ou au moins que la zone de contact du support d'assemblage (1) avec le corps de remplissage (10), ou inversement.
